# EUROPEAN PATENT APPLICATION

(11) **EP 1 207 395 A1**
(43) Date of publication of application: **22.05.2002**
(21) Application number: 00949981.5
(22) Date of filing: 03.08.2000
(51) Int. Cl.: G01N 33/573, G01N 33/50, G01N 33/15, C12Q 1/48, C07K 7/08, C07K 16/18

(54) **METHOD FOR MEASURING KINASE ACTIVITY OF CYCLIN/CDK COMPLEX**

(30) Priority: 04.08.1999 JP 22161299
(71) Applicant: Medical & Biological Laboratories Co., Ltd., Nagoya-shi, Aichi 460-0002 (JP)
(72) Inventor: SUZUKI, Susumu, Med. & Biol. Laborat. Co., Ltd., Ina-shi, Nagano 396-0002 (JP); TAMAI, Katsuyuki, Medic. & Biolog. Lab. Co., Ltd., Ina-shi, Nagano 396-0002 (JP); TOJI, Shingo, Medical & Biolog. Lab. Co., Ltd., Ina-shi, Nagano 396-0002 (JP); OGAWA, Akira, Takasaki-shi Gunma 370-1202 (JP)
(74) Representative: VOSSIUS & PARTNER
(86) International application number: JP0005219
(87) International publication number: WO0111367

(57) **Abstract**

The present invention provides methods for measuring the kinase activity of a cyclin/CDK complex on a RB protein, and methods for measuring the phosphatase activity of a cyclin/CDK complex on a RB protein phosphorylated by a cyclin/CDK complex, wherein the phosphorylation state of the RB protein to be phosphorylated by the cyclin/CDK complex is assessed using antibodies that can identify the phosphorylation state of the substrate. Compounds that regulate the activities of these enzymes can be screened by applying these measuring methods.

SEQUENCE LISTING

<110> Medical & Biological Laboratories CO., LTD

<120> METHOD FOR MEASURING KINASE ACTIVITY OF CYCLIN/CDK COMPLEX

<130> M3-104PCT

<140>
<141>

<150> JP 1999-221612
<151> 1999-08-04

<160> 20

<170> PatentIn Ver. 2.0

<210> 1
<211> 14
<212> PRT
<213> Homo sapiens

<220>
<223> Description of Artificial Sequence:Artificially
      Synthesized Peptide

<220>
<221> MOD_RES
<222> (7)
<223> PHOSPHORYLATION

<400> 1
Ser Phe Glu Thr Gln Arg Thr Pro Arg Lys Ser Asn Leu Cys
  1               5                  10

<210> 2
<211> 14
<212> PRT
<213> Homo sapiens

<220>
<223> Description of Artificial Sequence:Artificially
      Synthesized Peptide

<400> 2

Ser Phe Glu Thr Gln Arg Thr Pro Arg Lys Ser Asn Leu Cys
1               5                   10

<210> 3
<211> 14
<212> PRT
<213> Artificial Sequence

<220>
<221> MOD_RES
<222> (7)
<223> PHOSPHORYLATION

<220>
<223> Description of Artificial Sequence:Artificially
      Synthesized Peptide

<400> 3
Tyr Leu Ser Pro Val Arg Ser Pro Lys Lys Lys Gly Ser Cys
1               5                   10

<210> 4
<211> 14
<212> PRT
<213> Artificial Sequence

<220>
<223> Description of Artificial Sequence:Artificially
      Synthesized Peptide

<400> 4
Tyr Leu Ser Pro Val Arg Ser Pro Lys Lys Lys Gly Ser Cys
1               5                   10

<210> 5
<211> 14
<212> PRT
<213> Homo sapiens

<220>
<223> Description of Artificial Sequence:Artificially
      Synthesized Peptide

<220>

<221> MOD_RES
<222> (7)
<223> PHOSPHORYLATION

<400> 5
Thr Arg Pro Pro Thr Leu Ser Pro Ile Pro His Ile Pro Cys
1               5                   10

<210> 6
<211> 14
<212> PRT
<213> Artificial Sequence

<220>
<223> Description of Artificial Sequence:Artificially
      Synthesized Peptide

<400> 6
Thr Arg Pro Pro Thr Leu Ser Pro Ile Pro His Ile Pro Cys
1               5                   10

<210> 7
<211> 16
<212> PRT
<213> Homo sapiens

<220>
<223> Description of Artificial Sequence:Artificially
      Synthesized Peptide

<220>
<221> MOD_RES
<222> (7)
<223> PHOSPHORYLATION

<400> 7
Gly Gly Asn Ile Tyr Ile Ser Pro Leu Lys Ser Pro Tyr Lys Ile Cys
1               5                   10                  15

<210> 8
<211> 16
<212> PRT
<213> Homo sapiens

<220>
<223> Description of Artificial Sequence:Artificially
      Synthesized Peptide

<400> 8
Gly Gly Asn Ile Tyr Ile Ser Pro Leu Lys Ser Pro Tyr Lys Ile Cys
  1               5                  10                  15

<210> 9
<211> 30
<212> DNA
<213> Homo sapiens

<220>
<223> Description of Artificial Sequence:Artificially
      Synthesized Primer Sequence

<400> 9
ataggatcca tggaacacca gctcctgtgc                                   30

<210> 10
<211> 30
<212> DNA
<213> Homo sapiens

<220>
<223> Description of Artificial Sequence:Artificially
      Synthesized Primer Sequence

<400> 10
atactcgagg atgtccacgc tccgcacgtc                                   30

<210> 11
<211> 30
<212> DNA
<213> Homo sapiens

<220>
<223> Description of Artificial Sequence:Artificially
      Synthesized Primer Sequence

<400> 11
ataagatcta tgaaggagga cggcggcgcg                                   30

<210> 12
<211> 30

<212> DNA
<213> Homo sapiens

<220>
<223> Description of Artificial Sequence:Artificially
      Synthesized Primer Sequence

<400> 12
atactcgagc gccatttccg gcccgctgct                                        30

<210> 13
<211> 30
<212> DNA
<213> Homo sapiens

<220>
<223> Description of Artificial Sequence:Artificially
      Synthesized Primer Sequence

<400> 13
ataggatcca tggcgctccg agtcaccagg                                        30

<210> 14
<211> 30
<212> DNA
<213> Homo sapiens

<220>
<223> Description of Artificial Sequence:Artificially
      Synthesized Primer Sequence

<400> 14
atactcgagc acctttgcca cagccttggc                                        30

<210> 15
<211> 30
<212> DNA
<213> Homo sapiens

<220>
<223> Description of Artificial Sequence:Artificially
      Synthesized Primer Sequence

<400> 15
ataggatcca tggaagatta taccaaaata                                        30

<210> 16

<211> 30
<212> DNA
<213> Homo sapiens

<220>
<223> Description of Artificial Sequence:Artificially
      Synthesized Primer Sequence

<400> 16
atactcgaga ctcttcttaa tctgattgtc                                        30

<210> 17
<211> 30
<212> DNA
<213> Homo sapiens

<220>
<223> Description of Artificial Sequence:Artificially
      Synthesized Primer Sequence

<400> 17
ataggatcca tggagaactt ccaaaaggtg                                        30

<210> 18
<211> 30
<212> DNA
<213> Homo sapiens

<220>
<223> Description of Artificial Sequence:Artificially
      Synthesized Primer Sequence

<400> 18
atactcgagt cagagtcgaa gatggggtac                                        30

<210> 19
<211> 30
<212> DNA
<213> Homo sapiens

<220>
<223> Description of Artificial Sequence:Artificially
      Synthesized Primer Sequence

<400> 19
catggatcca tggctacctc tcgatatgag                                        30

<210> 20
<211> 30
<212> DNA
<213> Homo sapiens

<220>
<223> Description of Artificial Sequence:Artificially
      Synthesized Primer Sequence

<400> 20
catgtcgacc tccggattac cttcatcctt                                        30

## Description

### Technical Field

The present invention relates to methods for measuring the kinase or phosphatase activity of a cyclin/CDK complex, methods for screening cyclin/CDK complex inhibitors or enhancers, and kits used therefor.

### Background Art

During proliferation, cells periodically repeat the following processes: (1) replication of the genomic DNA, (2) distribution of the replicated DNA into daughter cells, and (3) cell division. These processes are collectively referred to as the cell cycle. The cell cycle can be divided into the G1 phase (DNA synthesis preparation phase); the S phase (DNA synthesis phase); the G2 phase (cell division preparation phase); and the M phase (cell division phase), and cells are divided by sequentially going through these phases. Progress of the cell cycle is precisely controlled by a number of molecules that suppress the undesirable proliferation of cells. Cloning and functional analyses of molecules related to the progress of the cell cycle have been frequently performed. Abnormalities in molecules related to the progress of the cell cycle have been reported in many cancers, and are presumed to be the cause of carcinogenesis.

For example, it has been elucidated that phosphorylation of RB proteins (retinoblastoma proteins) plays an important role in the progress of the cell cycle from the G1 phase to the S phase. The non-phosphorylated RB protein binds to E2F/DP, and suppresses the cell cycle, preventing progress to the S phase by inactivating the E2F/DP. The E2F/DP is a transcription factor that induces expression of genes, such as dihydrofolic acid reductase (DHFR) and DNA polymerase, that are essential for the progress to the S phase. The RB protein is phosphorylated by the action of cyclin D/CDK4, cyclin D/CDK6, or cyclin E/CDK2 and releases E2F/DP. A CDK (cyclin-dependent kinase) as used herein is an enzyme protein that expresses phosphorylation activity by forming a complex with cyclin. This means that the progress from the G1 phase to the S phase is controlled by the phosphorylation of the RB protein ("Cell cycle" In: Experimental Medicine Supplementary Editions BioScience Terminology Library, p62-93, 1995, published by Yodosha Co., LTD.).

The phosphorylation of the RB protein has been shown to be improperly controlled in many clinical cases of cancer. This is presumed to accelerate undesirable progress into the S phase, namely cell proliferation, which consequently appears to be related to carcinogenesis. For example, abnormal functioning of p53 (arising from a deletion or point mutation) leading to transcriptional induction of p21, an inhibitory factor of a cyclin/CDK complex, is observed with high incidence in various tumors. It is presumed that phosphorylation of the RB protein by a cyclin/CDK complex is abnormally enhanced because p21 is not produced normally in these clinical cases, which consequently leads to carcinogenesis. Further, deficiencies of inhibitory factors of the cyclin/CDK complexes, such as p15 and p16, have been also reported in many tumors. It is also considered that abnormal phosphorylation of the RB protein by a cyclin/CDK complex is a major cause of cancer formation in these cases. In addition, enhanced expression of cyclin D1 is also thought to be involved in carcinogenesis, for the same reason as described above.

Based on these backgrounds, a cyclin/CDK complex is used as a target molecule for developing anti-cancer drugs. Therefore, it is useful to develop a screening system for compounds that regulate the activity of a cyclin/CDK complex. To date, methods using radio-isotope (abbreviated as RI hereinafter) labeled compounds have been generally used for screening such compounds. However, since the use of RI is always accompanied with danger and disposal problems, screening systems which can be conducted without RI labels are desired.

In that vein, a simple and easy kinase activity assay for measuring PKA and PKC activity has been put to practical use. This method measures the phosphorylating activity of an enzyme by detecting enzymatic phosphorylation of a synthetic peptide called PS peptide, which is used as the substrate, with antibodies recognizing the phosphorylated PS peptide (T. Yano et al. Peptide Chemistry A. Suzuki (Ed): 293-298,1991). However, the PS peptide, which is the substrate peptide in this method, does not work as the substrate for cyclin/CDK complex, which is the kinase of RB proteins. In addition, the antibodies used in this method for detecting the level of phosphorylation cannot be used to measure the kinase activity of RB protein since it does not recognize the phosphorylated RB protein. Antibodies that recognize phosphorylated RB proteins are well known, but it is not known whether the kinase activity of the RB protein can be assayed by using these antibodies.

### Disclosure of the Invention

An object of the present invention is to provide methods for measuring the activity of cyclin/CDK complexes, enzymes that phosphorylate the RB protein, and methods for measuring the dephosphorylation activity of the RB protein phosphorylated by the action of the kinase. Another object of the present invention is to provide methods of screening for compounds that regulate the phosphorylation activity of a cyclin/CDK complex. Another object of the present invention is to provide methods of screening for compounds that regulate the kinase activity of the cyclin/CDK complexes using the RB protein as a substrate. A further object of the present invention is to provide methods that not only measure the kinase activity but also independently determine the respective phosphorylation activities of the cyclin/CDK complexes in a sample containing more than one cyclin/CDK complex. Yet a further object of the present invention is to provide a novel system that avoids the use of RI labeled compounds in the activity measuring and screening methods above. In addition, another object of the present invention is to provide kits for using the above measuring and screening methods.

To accomplish the objects above, the present inventors postulated that the phosphorylation activity of a cyclin/CDK complex may be detected using RB proteins as substrates to be phosphorylated by the cyclin/CDK complex, and by immunologically measuring the changes in the phosphorylation level of the RB proteins. The RB protein is suitable as the substrate protein, because the RB protein has been often reported to have relations to carcinogenesis as described above. The present inventors considered that, among the sites phosphorylated in the RB protein, the phosphorylation states of the threonine residue at position 356, the serine residue at position 612, the serine residue at position 780, and the threonine residue at position 807 may be particularly useful for assessing the phosphorylation activity of the cyclin/CDK complex. Accordingly, it was found that the phoshorylation levels of the substrate can be readily assessed using antibodies that identify the phosphorylation state of the site to be phosphorylated. In addition, the present inventors found that screening of compounds that inhibit or enhance phosphorylation or dephosphorylation of the RB protein could be achieved by measuring enzyme activity using such antibodies, and successfully completed the present invention.

Accordingly, the present invention provides methods for measuring the kinase activity or phosphatase activity of a cyclin/CDK complex, and methods for screening compounds that regulate the kinase or phosphatase activities of a cyclin/CDK complex based on these measuring methods. More specifically, the present invention provides the following:
(1) a method for measuring the kinase activity of a cyclin/CDK complex in a sample on RB proteins or partial peptides thereof, comprising the steps of:
   (a) contacting the RB protein substrate with the sample under conditions required for a phosphorylation reaction associated with the kinase to occur; and
   (b) detecting changes in the phosphorylation level of the RB protein substrate based on changes in reactivity of the substrate with antibodies that identify the phosphorylation state of the substrate;
(2) a method of screening for compounds that inhibit or enhance the kinase activity of a cyclin/CDK complex on an RB protein, comprising the steps of:
   (a) contacting and incubating the cyclin/CDK complex with the RB protein substrate in the presence of a test compound under conditions required for a phosphorylation reaction to occur;
   (b) detecting changes in the phosphorylation level of the RB protein substrate based on changes in reactivity of the substrate with antibodies that identify the phosphorylation state of the substrate; and
   (c) selecting the compound that lowers or elevates the phosphorylation level of the substrate as compared to the levels detected in the absence of the test compound;
(3) a method for measuring the phosphatase activity on phosphorylated RB proteins in a sample, comprising the steps of:
   (a) contacting and incubating the phosphorylated RB protein substrate with the sample under conditions required for a dephosphorylation reaction to occur; and
   (b) detecting changes in the phosphorylation level of the RB protein substrate based on changes in reactivity of the substrate with antibodies that identify the phosphorylation state of the substrate,
   wherein the phosphorylated RB protein is phosphorylated by a cyclin/CDK complex;
(4) a method of screening for compounds that inhibit or enhance the phosphatase activity on phosphorylated RB proteins, comprising the steps of:
   (a) contacting and incubating the phosphatase against the phosphorylated RB protein with the phosphorylated RB protein substrate in the presence of a test compound under conditions required for the dephosphorylation reaction;
   (b) detecting changes in the dephosphorylation level of the RB protein substrate based on changes in reactivity of the substrate with antibodies that identify the phosphorylation states of the substrate; and
   (c) selecting the compound that lowers or elevates the dephosphorylation level of the substrate compared to that in the absence of the test compound;
(5) the measuring method of any one of (1) to (4), wherein the cyclin is selected from the group consisting of: cyclin A, cyclin B, cyclin D1, cyclin D2, cyclin D3, and cyclin E;
(6) the measuring method of any one of (1) to (4), wherein the CDK is selected from the group consisting of: CDK 1, CDK 2, CDK 4, and CDK 6;
(7) the measuring method of any one of (1) to (4), wherein the cyclin/CDK complex comprises any one of the combinations selected from the group consisting of:
   cyclin A/CDK1;
   cyclin A/CDK2;
   cyclin B/CDK1;
   cyclin D1/CDK4;
   cyclin D1/CDK6;
   cyclin D2/CDK4;
   cyclin D2/CDK6;
   cyclin D3/CDK4;
   cyclin D3/CDK6; and
   cyclin E/CDK2;
(8) the method of (7), wherein the kinase is the cyclin D1/CDK4 complex, and the antibody identifies the phosphorylation state of the serine at position 780 of the RB protein;
(9) the method of (1), wherein individual activities of the cyclin/CDK complexes contained in a sample comprising a plurality of cyclin/CDK complexes that separately phosphorylate different sites can be measured, wherein the antibody that identifies the phosphorylation state is able to identify the phosphorylation site;
(10) the method of any one of (1) to (4), wherein the antibody has a higher reactivity with the phosphorylated RB protein substrate than with the non-phosphorylated RB protein substrate;
(11) the method of any one of (1) to (4), wherein the antibody has a higher reactivity with the non-phosphorylated RB protein substrate than with the phosphorylated RB protein substrate;
(12) the method of any one of (1) to (4), wherein the RB protein substrate is labeled;
(13) the method of any one of (1) to (4), wherein the RB protein substrate is immobilized on a solid phase;
(14) the method of any one of (1) to (4), wherein the antibody is labeled;
(15) the method of any one of (1) to (4), wherein the phosphorylation level of the RB protein substrate is evaluated by the ELISA method;
(16) a protein kinase activity regulating agent of the cyclin/CDK complex containing a compound, which can be isolated by the screening method described in (2) as the main principle;
(17) a protein phosphatase activity regulating agent of a protein phosphorylated by the kinase of a cyclin/CDK complex containing a compound, which can be isolated by the screening method described in (4) as the main principle;
(18) the activity regulating agent of (16) or (17), wherein the agent is derived from nature;
(19) an immunogen for preparing antibodies, which are used in any of the methods of (1) to (4), containing the RB protein or a peptide comprising an amino acid sequence including one or more of the phosphorylation sites of the homologues of the RB protein in species other than humans;
(20) the immunogen of (19), wherein the phosphorylation site(s) is(are) phosphorylated;
(21) the immunogen of (19) or (20), wherein the phosphorylation sites are selected from the group consisting of:
   threonine at position 356; serine at position 612; serine at position 780; and threonine at position 807 of the human RB protein;
(22) the immunogen of (21), wherein the phosphorylation site is selected from the group of amino acid sequences consisting of the sequence shown in SEQ ID NOs: 2, 4, 6, and 8; and
(23) a kit for performing any of the methods of (1) to (4),
wherein the kit contains an antibody that identifies the phosphorylation state of the RB protein substrate.

The term "peptide" herein refers to a compound in which more than two amino acids are ligated by peptide bonds, and there is no restriction to the chain length of the peptide. Thus, proteins are also included in the term "peptide" according to the present invention.

Cyclins constituting the cyclin/CDK complexes, which is the object of measurements in the present invention, include, for example, cyclin A, cyclin B, cyclin D1, cyclin D2, cyclin D3, and cyclin E. CDKs known in the art include, CDK1, CDK2, CDK4, and CDK6. Amino acid sequences constituting these proteins, and DNA nucleotide sequences encoding the proteins are well known in the art. Combinations of CDK and cyclin known in the art include the following; however, the cyclin/CDK complex of the present invention is not limited to one of these known combinations:
cyclin A/CDK1;
cyclin A/CDK2;
cyclin B/CDK1;
cyclin D1/CDK4;
cyclin D1/CDK6;
cyclin D2/CDK4;
cyclin D2/CDK6;
cyclin D3/CDK4;
cyclin D3/CDK6; and
cyclin E/CDK2

Cyclin and CDK constituting the complex may be naturally occurring enzymes derived from human tissues, as well as recombinant proteins obtainable by expressing a gene encoding them in an appropriate expression system. The origins of the enzymes are not restricted to humans, and enzymes may be homologues of other vertebrates and microorganisms. Furthermore, not only enzymes having the same amino acid sequence as a naturally occurring enzyme protein, but also enzyme proteins having essentially the same activity as the enzyme described above, for example, mutants composed only of the region supporting the expression of the enzyme activity (a kinase region), or a fusion protein prepared by fusion of this region with other proteins, may be used as the objects for measuring the kinase activity according to the present invention.

In a first aspect, the present invention features a method for measuring the kinase activity by using antibodies that specifically bind to an RB protein substrate, which is phosphorylated by a cyclin/CDK complex. Specifically, the measuring method comprises the steps of:
(a) contacting the RB protein substrate with the sample under a condition required for a phosphorylation reaction associated with the kinase to occur; and
(b) detecting changes in the phosphorylation level of the RB protein substrate based on the changes in reactivity of the substrate with the antibody that can identify the phosphorylation states of the substrate.

The term "RB protein substrate" in the present invention refers to human RB proteins, homologues of the RB protein in other species, and partial peptides containing at least the site to be phosphorylated in these proteins. The term "the site to be phosphorylated" refers to an amino acid sequence consisting of three amino acid residues including at least one amino acid before and after the amino acid to be phosphorylated. The RB protein, or its homologue which is used as the substrate, may be any one selected from the group of naturally occurring proteins, proteins prepared using gene recombination technology, and synthetic peptides. Structures of the RB protein (Nature 329, 6140, 642-645, 1987; GenBank Acc. No. M28419), and homologues thereof, p107 (Cell 66, 1155-1164, 1991; Genes Dev. 7/7A, 1111-1125, 1993; GenBank Acc. No. L14812) and p130 (Genes Dev. 7/12A, 2366-2377, 1993; GenBank Acc. No. X76061), are known in the art, and the DNA nucleotide sequences encoding them are also known. The peptides may be fused with other peptides (for example glutathione-S-transferase) to facilitate the purification of the peptides. The RB protein substrate is used in a sufficient amount relative to the expected enzyme activity. For example, if almost all of the substrate is phosphorylated in a phosphorylation reaction under a certain substrate condition, there is a possibility that the substrate may have run short. In this case, it is desirable to increase the substrate concentration, or increase the relative concentration of the substrate to the kinase activity by diluting the sample and such.

Peptide fragments composed of partial amino acid sequences containing the phosphorylation region of the RB protein or homologues thereof may be used as the RB protein substrate. For example, a peptide containing any one of the amino acid sequences indicated in SEQ ID NOs: 2, 4, 6 or 8 selected from the amino acid sequence of the human RB protein may be used as the RB protein substrate. A peptide in which the phosphorylation site of these peptides has been phosphorylated may be also used as the phosphorylated peptide in the present invention.

According to the present invention, the RB protein substrate is contacted with the sample under conditions that allow phosphorylation of the substrate by a cyclin/CDK complex. "Conditions that allow phosphorylation" means that the incubation is conducted in a reaction media which gives a pH necessary for the maintenance of the subject cyclin/CDK complex activity, in the presence of components necessary for the reaction, along with the RB protein substrate at a proper reaction temperature. Substrates providing a phosphate group necessary for the phosphorylation reaction, a phosphatase inhibitor that protects the phosphorylation product, and so on can be given as the components "necessary for the reaction". In general, adenosine triphosphate (ATP) and the like are used as the substrates providing phosphate groups, and β-glycerophosphoric acid and the like are used as the phosphatase inhibitors. A reaction buffer for cyclin/CDK that consists of the following composition can be given as a buffer for the phosphorylation reaction in the present invention.

### Reaction buffer for cyclin/CDK:

50 mM HEPES
15 mM MgCl₂
1 mM DTT
0.02% Trion X
1 mM EGTA
5 mM ATP

According to the present invention, the phosphorylated level of a phosphorylated RB protein substrate is evaluated by an immunological reaction with antibodies that can identify the phosphorylation state of the substrate. The term "the level of phosphorylation" herein refers not only to the ratio of the phosphorylated RB protein substrate to the whole RB protein substrate, but also to the degree of phosphorylation of each RB protein substrate. Therefore, for example, the increase of the phosphorylation level of the RB protein substrate means either the state wherein the ratio of the phosphorylated substrate is increased, or the state wherein the number of phosphorylated sites on a substrate is increased.

The antibodies used in the present invention that can identify the phosphorylation state comprise antibodies whose reactivity with the RB protein substrate changes according to the presence or absence of the phosphorylation. More specifically, antibodies having higher (or lower) reactivity to a phosphorylated RB protein substrate than to the substrate in an unphosphorylated state can be exemplified. Antibodies which can identify the phosphorylated state of the RB protein substrate are well-known in the art (S-Y. Shieh et al. Cell Vol 91, p325, 1997). Similar antibodies can be prepared using methods well-known to one skilled in the art (for example, Cell Technology, separate volume, anti-peptide antibody experiment protocol, 1994, Shujunsha; B. M. Turner and G. Fellows, Eur. J. Biochem. 179, 131-139, 1989; S. Muller et al. Molecular Immunology Vol 24, 779-789, 1987; Pfeffer, U., Ferrari, N. and Vidali, G., J. Biol. Chem. 261, 2496-2498, 1986). The antibody may be a monoclonal antibody or a polyclonal antibody.

For example, polyclonal antibodies specific to a phosphorylated RB protein substrate can be purified from antiserum obtained by immunizing an animal with the phosphorylated peptide, using an unphosphorylated peptide-immobilized column (non-specific antibody absorption column) or phosphorylated peptide-immobilized column (specific antibody purification column). Alternatively, monoclonal antibody producing hybridoma can be cloned by screening hybridomas producing specific antibodies from those established from antibody producing cells of animals immunized with the phosphorylated peptide. The screening involves examining the reactivity of the culture supernatant to unphosphorylated and phosphorylated peptide by the ELISA method, and selecting clones with a high reactivity against the phosphorylated peptide. The desired monoclonal antibody can be purified from the culture supernatant or ascites by culturing the cloned hybridoma.

Immunogens for preparing antigen to be used in the present invention can be produced using a peptide containing the amino acid sequence constituting the phosphorylation site of the human RB protein. Accordingly, for example, regions containing partial amino acid sequences having the phosphorylation site of the human RB protein described in SEQ ID NOs: 2, 4, 6 or 8 can be selected as the immunogen of the present invention. At a minimum, the phosphorylation site consists of three amino acid residues - at least one amino acid before and after the amino acid to be phosphorylated. The amino acid sequence of the peptide constituting the immunogen of the present invention is not restricted to the amino acid sequence of the human RB protein. More specifically, regions containing the amino acid sequence constituting the phosphorylation site from the amino acid sequence of a homologue of the RB protein in other species may be selected as the immunogen of the present invention. p107 and p130 are known homologues of the RB protein. Antibodies that can identify the phosphorylation state of the human RB protein can be obtained using the immunogen prepared based on the amino acid sequence of a homologue.

Reactivity of the antigen is affected by the state of phosphorylation at the phosphorylation site of the peptide which is used as the immunogen. More specifically, antibodies having high reactivity against the phosphorylated RB protein can be obtained by utilizing a phosphorylated peptide, and conversely, antibodies having high reactivity against the non-phosphorylated RB protein can be obtained when the peptides that are not phosphorylated are used. The peptides constituting the immunogen can be obtained as chemically synthesized peptides, gene recombinants, naturally occurring RB proteins or homologues thereof, and fragments thereof. Methods for selectively. phosphorylating these peptides at desired sites are also known in the art. Immunogenicity of the peptide can be enhanced by covalently binding the peptides with a carrier protein. Keyhole limpet hemocyanin and such may be generally used as the carrier protein. Cross-linking substances available for forming a covalent bond with the carrier protein include m-maleimide benzoyl-N-hydroxysuccinimide ester. Adjuvants known in the art may be formulated with the immunogen obtained as described above.

As a desirable embodiment, the present invention provides measuring methods, wherein not only the phosphorylation states can be identified but also wherein the structural differences, including the surrounding structures of the phosphorylation site of the RB protein, can be identified using antibodies. A cyclin/CDK complex does not always phosphorylate the same site of the RB protein substrate. For example, phosphorylation of the cyclin D1/CDK4 complex mainly proceeds at the serine residues at positions 780 and 795, and at the threonine residue at position 811. On the other hand, the sites mainly phosphorylated in the cyclin B/CDK2 are the threonine residues at positions 356 and 821. Specificity of both cyclin/CDK complexes are listed in Table 1. The amino acids are identified herein by indicating the position of the amino acid residue counted from the N-terminal of the RB protein after the code (represented by one or three capital letters) denoting an amino acid. For example, S780 denotes a serine at position 780.

**Table 1**

| | kind of cyclin/CDK complex | | |
|---|---|---|---|
| phosphorylation site | | | |
| | D1/CDK4 | E/CDK2 | B/CDK2 |
| T356 | - | +++ | ++ |
| T373 | + | + | - |
| S608 | ++ | | |
| S612 | - | +++ | - |
| S780 | +++ | - | - |
| S795 | +++ | + | - |
| T807 | ++ | - | - |
| T811 | +++ | ++ | - |
| T821 | - | +++ | ++ |
| T826 | - | ++ | - |

Accordingly, the kinase activity of a cyclin D1/CDK4 complex can be specifically measured using antibodies that identify the phoshorylation state of serine at position 780. The phosphorylation by the cyclin D1/CDK4 complex and phosphorylation by the cyclin B/CDK2 can be separately assessed by utilizing antibodies that specifically recognize the phosphorylation sites. Thus, the present invention provides measuring methods wherein individual phosphorylation activities can be separately determined even when using samples containing several kinds of cyclin/CDK complexes.

The reaction of the RB protein substrate and the antibody can be detected by following conventional immunoassay principle. In general, the separation of unreacted components can be easily achieved by immobilizing either the RB protein substrates or antibodies to a solid phase. Methods to immobilize proteins, peptides, or antibodies to a solid phase are well-known in the art. For example, the RB protein substrates and antibodies can be directly immobilized onto a solid phase by chemical bonding and physical adsorption. Alternatively, a biotinylated RB protein substrate can be indirectly immobilized on a solid-phased sensitized with streptavidin. In general, the immobilization of an RB protein substrate to a solid-phased is conducted under conditions wherein the adsorption of the protein to the carrier is saturated. In this case, the immobilization of the biotinylated RB protein substrate to the solid-phase is arbitrary, and may be conducted before or after contacting with the sample, simultaneously with the contact, and so on. In addition, systems other than the avidin-biotin system can be applied to the present invention so long as it is a combination of substances having mutual affinity. For example, the RB protein substrate can be prepared as a conjugate with an appropriate antigenic material, and the RB protein substrate may be captured using solid phase sensitized with antibodies against the antigenic material.

Generally, used solid phases, such as the inner wall of a reaction container, granular carriers, and bead-shaped carriers, can be used as the solid phase for immobilizing the RB protein substrate or antibody of the present invention. The material of the solid-phase may be those generally used for physically adsorbing or chemically binding proteins. More specifically, a microtiter plate made of polystyrene, or the like, can be used.

When changes in the phosphorylation level are evaluated based on the immunoassay principle of the present invention, simple and easy operation can be achieved by labeling either the RB protein substrate or the antibody. There is no limitation on the label to be used, so long as it has a sensitivity sufficient enough for detection. For example, enzyme labels such as peroxidase, β-D-galactosidase, alkaline phosphatase, glucose-6-phosphate dehydrogenase, and acetylcholinesterase; fluorescent labels such as delphinium; radiolabels; and such may be used. Moreover, not only direct labeling, but also the so-called indirect labeling system can be used, wherein a material that binds specifically to the antibody, for example, a secondary antibody, protein A, protein G, protein A/G (fusion protein of protein A and G), and such are labeled.

The phosphorylation level of the RB protein substrate can be evaluated according to the specificity of the antibody and the label used by methods well-known to one skilled in the art (for example, see "Super-high sensitivity enzyme immunoassay method" Ishikawa E., Japan Scientific Societies Press (1993)). Namely, when a combination of an antibody reacting with a phosphorylated RB protein and a labeled RB protein is used, the amplitude of the signal originating from the labeled RB protein substrate that reacted with the antibody is proportional to the phosphorylation activity. On the other hand, when an antibody reactive to an unphosphorylated RB protein substrate is used, the amplitude of the signal is inversely proportional to the phosphorylation activity. Phosphorylation levels can be quantified based on a calibration curve, which is made beforehand using a RB protein substrate of known phosphorylation level as the standard sample. Alternatively, a qualitative comparison can be conducted using a sample free of the kinase activity as the control.

Any sample predicted to contain cyclin/CDK complex can be used as the target of the method for measuring the kinase activity of the present invention. Tissue samples derived from humans and non-human animals; extracts of various cultured cells; culture supernatants of cells; secretions such as blood, urine, body fluids, sweat, saliva, and milk; and so on can be given as typical samples.

Furthermore, the present invention provides a method for measuring not only the kinase activity, but also the phosphatase activity of a cyclin/CDK complex on the RB protein substrate phosphorylated . The method for measuring the phosphatase activity of this invention comprises the steps of:
(a) contacting and incubating a sample with a phosphorylated RB protein substrate under conditions necessary for the dephosphorylation reaction to occur; and,
(b) detecting changes in the phosphorylation level of the substrate based on changes in reactivity of the substrate with antibodies that identify the phosphorylation state of the substrate.

The phosphorylation of the RB protein substrate is achieved by treating the RB protein with cyclin/CDK complex, under conditions described above, so that phosphorylation can take place.

The same antibodies as described above can be used as antibodies that can identify the phosphorylation state of the RB protein substrate used in the measurement of the phosphatase activity. Moreover, the method for evaluating the phosphorylation level of the RB protein substrate based on immunoassay can be also conducted based on a similar principle as described above. The only difference is that the relationship between the amplitude of the signal derived from the labeled RB protein substrate (or labeled antibody) in the measurement of the phosphatase activity and the activity of the enzyme to be measured is inversed.

To date, no proteins have been identified as proteins functioning in the human living body as a phosphatase against a protein phosphorylated by a cyclin/CDK complex. However, the method of this invention for measuring the phosphatase activity can serve as an assay system that enables the measurement of the total phosphatase activity comprised in the tissue or cell extract, and such.

Various samples suspected to contain the phosphatase activity can be used as target of the method for measuring the phosphatase activity of the present invention, in a manner analogous to the method for measuring the kinase activity. For example, when one attempts to purify a phosphatase contained in an extract, the phosphatase activity detected in each fraction is useful as an index of enzyme purification.

Methods for measuring the kinase activity or the phosphatase activity of the present invention can be used for screening inhibitors and accelerators of the kinase or phosphatase, respectively. Thus, the present invention relates to methods of screening for compounds that inhibit or accelerate the activity of a kinase and phosphatase. Compounds that inhibit or accelerate these enzyme activities are generally designated as "compounds that regulate enzyme activity" in the present invention.

A method of screening for compounds that inhibit or accelerate the kinase activity of this invention comprises the steps of:
(a) contacting and incubating the cyclin/CDK complex with the RB protein substrate in the presence of a test compound under conditions necessary for the phosphorylation reaction to occur;
(b) detecting changes in the phosphorylation level of the RB protein substrate based on changes in the reactivity of the substrate peptide with antibodies that have the ability to identify the phosphorylation state of the substrate; and,
(c) selecting compounds that decreases or increases the change in the phosphorylation level of the substrate by comparing with the phosphorylation level of the substrate detected or observed in the absence of the test compound.

The contact of the cyclin/CDK complex and the RB protein substrate, as well as the evaluation of the phosphorylation level of the RB protein substrate, can be conducted by methods similar to those used in the measurement of the kinase activity described above. As a result, a test compound used in the screening is judged to accelerate the kinase activity when the detected amount of phosphate group(s) binding to serine or threonine residue(s) of the RB protein substrate is decreased in the absence of the test compound.

Not only naturally occurring cyclin/CDK complexes, but also enzyme proteins that can be obtained as recombinant proteins can be used as the kinase used in the screening methods of the present invention. Moreover, not only enzymes comprising the same amino acid sequence as the natural enzyme, but also enzyme fragments consisting of only the kinase domain can be used as well. The size of the expression product can be made smaller by using only the protein kinase domain, and as a result, a larger amount of enzyme protein can be easily obtained as a recombinant protein.

The method of screening for compounds that inhibit or accelerate the phosphatase activity of the present invention comprises the steps of:
(a) contacting and incubating a phosphatase of the phosphorylated RB protein with the phosphorylated RB protein substrate in the presence of a test compound under conditions necessary for the dephosphorylation reaction to occur,
(b) detecting changes in the dephosphorylation level of the RB protein substrate based on changes in the reactivity of the substrate with antibodies that have the ability to identify the phosphorylation state of the substrate peptide; and,
(c) selecting compounds that decrease or increase the changes in the dephosphorylation level of the substrate by comparing with the dephosphorylation level of the substrate detected or observed in the absence of the test compound.

For example, peptides (including proteins); synthetic low molecular compounds; cellular extracts of animals, plants, and bacteria; cell culture supernatants; and such can be used as the test compound of these screening methods, but the invention is not limited thereto. Moreover, for example, *E. coli* alkaline phosphatase; calf intestinal alkaline phosphatase; phosphatases such as Cdc25 and Cdc14; and so on can be used as the phosphatase.

The contact of the phosphatase and phosphorylated RB protein substrate, and the evaluation of the phosphorylation level of the RB protein substrate can be conducted by methods similar to those used for measuring the phosphatase activity described above. As a result, the test compound used in the screening is judged to inhibit the phosphatase activity when an increase in the amount of the phosphate group(s) binding to serine and/or threonine residue(s) of the RB protein is detected, compared with the detected amount of phosphate group(s) binding to serine and/or threonine residue(s) of the RB protein substrate in the absence of the test compound (control). On the contrary, the test compound used in the screening is judged to accelerate phosphatase activity when a decrease in the amount of the phosphate group(s) that binds to serine and/or threonine residue(s) of the RB protein is detected.

When cell extracts of animals, plants, and bacteria, cell culture supernatants, and such are used as the test compound in the screening method of the present invention, a single compound which inhibits the kinase (or phosphatase) activity can be finally specified by fractionating these test compounds by methods well known to one skilled in the art (for example, various chromatography methods) and detecting kinase or phosphatase activities in each fraction. The compounds that inhibit or enhance activities of phosphorylation and phosphatases isolated by the screening of the present invention may be utilized as activity regulating agents of these enzymes. These compounds are particularly useful as candidates drugs for treating cancer and rheumatism. When the compounds isolated by the screening method of the present invention are used as activity regulating agents for the kinase activity or phosphatase activity, they may be formulated according to known pharmaceutical methods. For example, the compound may be administered to a patient together with pharmacologically acceptable carriers or media (such as physiological saline, plant oil, suspending agents, surfactants, and stabilizers). They can be administered percutaneously, intranasally, transbronchially, intramusculary, intravenously, or orally, depending on the properties of the compounds. Although the dosage changes according to the age, weight, and clinical conditions of a patient, administration methods, and such, but those skilled in the art can appropriately select them.

Additionally, the present invention relates to a kit comprising an antibody that can identify the phosphorylation state of the RB protein substrate, wherein such a kit may be used in the assay or screening method described above. The kit of the present invention, used for the detection of kinase activity, comprises, for example, an RB protein substrate and buffer in addition to the above-mentioned antibody. On the other hand, when the kit is used for the detection of phosphatase activity, it comprises at a minimum a phosphorylated RB protein substrate and buffer in addition to the above-mentioned antibody. Whereas a kinase (or phosphatase) is further combined to the kit, when using it for the screening of compounds that inhibit or promote the activity of these enzymes. Either of the RB protein substrate or the above-mentioned antibody can be labeled as mentioned above, while the other can be immobilized on a solid-phase.

Standard enzyme(s) and/or RB protein substrate sample(s) can be combined in the kits for the calibration of the kinase (or phosphatase) activity and/or the assay system itself. Other components for stabilization, and such, can be added to these standard samples and above-mentioned antibodies. For example, 1% of BSA, and polyols such as sucrose and fructose with a final concentration of 0.2%∼10% (preferably 1%) can be added to the standard sample as a protein degeneration inhibitor after freeze-drying. The present invention will be explained in detail below with reference to Examples.

### Brief Description of the Drawings

Figure 1 shows graphs demonstrating measurement results of the phosphorylation reactions at each site on the RB protein by the ELISA method. The name of each monoclonal antibody used in the measurement, and the phosphorylation site recognized by the monoclonal antibody are shown in each graph. In the graphs, D1/4 denotes the combination of cyclin D1/CDK4; E/2 the combination of cyclin E/CDK2; A/2 the combination of cyclin A/CDK2; and B/1 the combination of cyclin B/CDK1.
Figure 2 shows a graph demonstrating the inhibitory effects of inhibitors K252a (K), Olomoucine (O), and Roscovitine (R) on the cyclin B/CDK1 complex measured by the ELISA method.
Figure 3 shows a graph demonstrating the inhibitory effects of inhibitors K252a (K), Olomoucine (O), and Roscovitine (R) on cyclin A/CDK2 complex measured by the ELISA method.
Figure 4 shows a graph demonstrating the inhibitory effects of inhibitors K252a (K), Olomoucine (O), and Roscovitine (R) on cyclin E/CDK2 complex measured by the ELISA method.
Figure 5 shows a graph demonstrating the inhibitory effects of inhibitors K252a (K), Olomoucine (O), and Roscovitine (R) on cyclin D1/CDK4 complex measured by the ELISA method.
Figure 6 shows a graph demonstrating the inhibitory effects of inhibitors Olomoucine-isomer (Oi) and U0126 (U) on cyclin B/CDK1 complex measured by the ELISA method.
Figure 7 shows a graph demonstrating the inhibitory effects of inhibitors Olomoucine-isomer (Oi) and U0126 (U) on cyclin A/CDK2 complex measured by the ELISA method.
Figure 8 shows a graph demonstrating the inhibitory effects of inhibitors Olomoucine-isomer (Oi) and U0126 (U) on cyclin E/CDK2 complex measured by the ELISA method.
Figure 9 shows a graph demonstrating the inhibitory effects of inhibitors Olomoucine-isomer (Oi) and U0126 (U) on cyclin D1/CDK4 complex measured by the ELISA method.
Figure 10 shows the comparative results of sensitivity between the ELISA method and RI method. The solid line and broken line denote the results of the ELISA method and RI method, respectively.
Figure 11 shows the comparative results of the inhibitory effects of the inhibitor Olmoucine on activities of cyclin/CDK complexes measured by the ELISA method and RI method.
Figure 12 shows the comparative results of the inhibitory effects of the inhibitor Roscovitine on activities of cyclin/CDK complexes measured by the ELISA method and RI method.
Figure 13 shows the comparative results of the inhibitory effects of the inhibitor Olmoucine-isomer on activities of cyclin/CDK complexes measured by the ELISA method and RI method.

### Best Mode for Carrying out the Invention

### [EXAMPLE 1] Preparation of anti-phosphorylated peptide antibody

### (1) Preparation of immunogen

### (a) Preparation of peptide

Eight peptides containing threonine at position 356, serine at position 612, serine at position 780, and serine at position 807 of human RB protein, respectively, which positions are reported as the phosphorylation sites, were produced as follows using a peptide synthesizer:

phospho-pRB-T356:  SFETQRT(p)PRKSNLC (SEQ ID NO: 1)
pRB-T356:  SFETQRTPRKSNLC (SEQ ID NO: 2)
phospho-pRB-S612:  YLSPVRS(p)PKKKGSC (SEQ ID NO: 3)
pRB-S612:  YLSPVRSPKKKGSC (SEQ ID NO: 4)
phospho-pRB-S780:  TRPPTLS(p)PIPHIPC(SEQ ID NO: 5)
pRB-S780:  TRPPTLSPIPHIPC (SEQ ID NO: 6)
phospho-pRB-S807: GGNIYIS(p)PLKSPYKIC (SEQ ID NO: 7)

pRB-S807: GGNIYISPLKSPYKIC (SEQ ID NO: 8)

Each amino acid in the sequences above is expressed by one capital letter, and the sequence is arranged so that the amino terminal is on the left of the sheet and the carboxyl terminal on the right. S(p) and T(p) denote a phosphorylated serine residue and phosphorylated threonine residue, respectively. Each peptide was confirmed to have a purity of 90% or more by HPLC. Phospho-pRB-T356 corresponds to an amino acid sequence from the 350th to 362nd amino acid, phospho-pRB-S612 corresponds to an amino acid sequence from the 606th to 618th amino acid, phospho-pRB-S780 corresponds to an amino acid sequence from the 774th to 786th amino acid, and phospho-pRB-S807 corresponds to an amino acid sequence from the 801st to 815th amino acid of the human RB protein. All the cysteine residues at the carboxyl terminus were introduced into the peptides in order to bind the peptides to a carrier protein by a covalent bond.

### (b) Bonding of peptides to a carrier protein

Each of the phosphorylated peptides (phospho-pRB-T356, -S612, -S780, and -S807) was bound to keyhole limpet hemocyanin (KLH), the carrier protein, through covalent bonds. The m-maleimide benzoyl-N-hydroxysuccinimide ester (MBS) was used as a crosslinking agent to bind these peptides to KLH. The peptides were cross-linked with equivalent KLH, and this peptide-KLH was used as an immunogen.

### (c) Preparation of immunogen and immunization method

10 µg carrier protein KLH together with a peptide bound thereto (peptide-KLH; 100 µl) was used as an immunogen for one challenge to a mouse. Freund's complete adjuvant (100 µl) and peptide-KLHs (100 µl each) were mixed to from a complete emulsion using a 1 ml syringe with a No. 21 gage injection needle in a 1.5 ml tube.

The emulsion of each phosphorylated peptide with the adjuvant was injected into the abdominal cavity of a mouse (Balb/c) using a No. 26 gage injection needle. The mouse was immunized once a week with four challenges in total. 50 to 100 µl of blood was extracted from the orbital plexus venosus after the fourth challenge in order to check the antibody titer by the ELISA method.

### (2) Production of monoclonal antibodies against phospho-pRB-T356, phospho-pRB-S612, phospho-pRB-S780, and phospho-pRB-S807

### (a) Preparation of hybridoma

After confirming a sufficient antibody titer, spleen were removed from the mice and spleen cells were fused with myeloma cells using polyethylene glycol. Selection of hybridomas was carried out by the HAT (hypoxanthine, aminopterin, thymidine) selection. Clones having a higher reactivity to phosphorylated peptides (phospho-pRB-T356, phospho-pRB-S612, phospho-pRB-S780, and phospho-pRB-S807) than to unphosphorylated peptides (pRB-T356, pRB-S612, pRB-S780, and pRB-S807) were screened by ELISA using the culture supernatants of hybridomas.

### (b) Obtaining ascites and purification of the antibodies

Hybridoma clones obtained by limiting dilution was cultured in 75 cm² flasks, and were injected intraperitoneally to mice. 1 ml and 0.5 ml of pristane (Sigma) was injected to mice 10 days and 3 days before injecting the hybridoma, respectively. One or two weeks later, the ascites were collected according to the observed condition of the mice. Ammonium sulfate was added to the ascites to a final concentration of 50%, and the solution was stirred for more than 1 hour at 4°C. The precipitate was collected by high-speed centrifugation. After completely dissolving the precipitate with a minimum amount of pure water, it was dialyzed against PBS using a dialysis membrane. After completely equilibrating to PBS, complexes with protein A sepharose (Pharmacia) were formed. Finally, the antibody was eluted from protein A sepharose.

### (3) Antibody titer and specificity testing

The peptide was dissolved in PBS to a concentration of 10 µg/ml, and 50 µl thereof were seeded to each well of a microtiter plate for ELISA, and were sensitized overnight at 4°C. After sensitization, the peptide solution was removed, 200 µl of 1% BSA-0.1% Tween 20-PBS was added to each well, and was blocked for more than 1 hour at room temperature.

Purified antibodies were diluted with 0.1% Tween 20-PBS according to needs. The diluted samples were added to the wells of the sensitized plate at 100 µl/well, and was left standing for 1 hour (primary reaction). After the primary reaction, each well was washed thoroughly more than 4 times with PBS using a wash-bottle. Horseradish peroxidase (HRP)-labeled goat anti-mice IgG (H+L) antibody (MBL, 330) diluted 3000-fold with 0.1% Tween 20-PBS was seeded at 100 µl/well, and the plate was left standing for 1 hour at room temperature (secondary reaction). After the secondary reaction, the wells were washed in the same way with PBS, 100 µl of 750 µM TMB (Tetramethylbenzidine) solution was added to each well, and was left to develope colors for 5 to 20 minutes at 30°C (color reaction) . The color reaction was quenched by adding 100 µl/well of 1.5 N H₃PO₄, and the absorbance at 450 nm was measured using a microtiter plate reader.

### [EXAMPLE 2] Preparation of recombinant cyclin D1/CDK4, cyclin E/CDK2, and cyclin B/CDK1

### (1) cDNA isolation of cyclin and CDK by the PCR method

### (a) Preparation of respective cyclin and CDK amplification primers

Primers as shown below were prepared in order to amplify full-length cDNAs of each cyclin and CDK. Nucleotide sequences of the cDNAs of each cyclin and CDK are registered in the GenBank with the following accession numbers:
cyclin D1: X59798
cyclin E: M73812, M74093
cyclin B: M25753
CDK1: X05360
CDK2: X61622, X62071
CDK4: NM_000075

### 〈Cyclin D1 amplification primer〉

Forward primer (hcy-D1 F1): 5'-ATA GGA TCC ATG GAA CAC CAG CTC CTG TGC-3' (SEQ ID NO: 9) (The three bases (ATA) at the 5'-end facilitate the treatment with restriction enzymes. Bases from the fourth to the ninth base from the 5'-end (GGA TCC) serve as a restriction enzyme Bam HI sitè.)

Reverse primer (hcy-D1 R1): 5'-ATA CTC GAG GAT GTC CAC GCT CCG CAC GTC-3' (SEQ ID NO: 10) (The three bases from the 5'-end (ATA) facilitate the treatment with restriction enzymes. Bases from the fourth to ninth base from the 5'-end (CTC GAG) serve as a restriction enzyme Xhol I site.)

### 〈Cyclin E amplification primer〉

Forward primer (hcy-E F1): 5'-ATA AGA TCT ATG AAG GAG GAC GGC GGC GCG-3' (SEQ ID NO: 11) (The three bases (ATA) at the 5'-end facilitate the treatment with restriction enzymes. Bases from the fourth base to ninth base from the 5'-end (AGA TCT) serve as a restriction enzyme Bgl II site.)

Reverse primer (hcy-E R1): 5'-ATA CTC GAG CGC CAT TTC CGG CCC GCT GCT-3' (SEQ ID NO: 12) (The three bases from the 5'-end (ATA) facilitate the treatment with restriction enzymes. Bases from the fourth base to ninth base from the 5'-end (CTC GAG) serve as a restriction enzyme Xho I site.)

### 〈Cyclin B amplification primer〉

Forward primer (hcy-B F1): 5'-ATA GGA TCC ATG GCG CTC CGA GTC ACC AGG-3' (SEQ ID NO: 13) (The three bases (ATA) at the 5'-end facilitate the treatment with restriction enzymes. Bases from the fourth base to ninth base from the 5'-end (CTC GAG) serve as a restriction enzyme Xho I site.)

Reverse primer (hcy-B R1): 5'-ATA CTC GAG CAC CTT TGC CAC AGC CTT GGC-3' (SEQ ID NO: 14) (The three bases from the 5'-end (ATA) facilitate the treatment with restriction enzymes. Bases from the fourth base to ninth base from the 5'-end (CTC GAG) serve as a restriction enzyme Xho I site.)

### 〈CDK1 amplification primer〉

Forward primer (hCDK1 F1): 5'-ATA GGA TCC ATG GAA GAT TAT ACC AAA ATA-3' (SEQ ID NO: 15) (The three bases (ATA) at the 5'-end serve facilitate the treatment with restriction enzymes. Bases from the fourth base to ninth base from the 5'-end (GGA TCC) serve as a restriction enzyme Bam HI site.)

Reverse primer (hCDK1 R1): 5'-ATA CTC GAG ACT CTT CTT AAT CTG ATT GTC-3' (SEQ ID NO: 16) (The three bases from the 5'-end (ATA) facilitate the treatment'with restriction enzymes. Bases from the fourth base to ninth base from the 5'-end (CTC GAG) serve as a restriction enzyme Xho I site.)

### 〈CDK2 amplification primer〉

Forward primer (hCDK2 F1): 5'-ATA GGA TCC ATG GAG AAC TTC CAA AAG GTG-3' (SEQ ID NO: 17) (The three bases (ATA) at the 5'-end facilitate the treatment with restriction enzymes. Bases from the fourth base to ninth base from the 5'-end (GGA TCC) serve as a restriction enzyme Bam HI site.)

Reverse primer (hCDK2 R1): 5'-ATA CTC GAG TCA GAG TCG AAG ATG GGG TAC-3' (SEQ ID NO: 18) (The three bases from the 5'-end (ATA) facilitate the treatment with restriction enzymes. Bases from the fourth base to ninth base from the 5'-end (CTC GAG) serve as a restriction enzyme Xho I sites.)

### 〈CDK4 amplification primer〉

Forward primer (hCDK4 F1): 5'-CAT GGA TCC ATG GCT ACC TCT CGA TAT GAG-3' (SEQ ID NO: 19) (The three bases (CAT) at the 5'-end facilitate the treatment with restriction enzymes. Bases from the fourth base to ninth base from the 5'-end (GGA TCC) serve as a restriction enzyme Bam HI site.)

Reverse primer (hCDK4 R1): 5'-CAT GTC GAC CTC CGG ATT ACC TTC ATC CTT-3' (SEQ ID NO: 20) (The three bases from the 5'-end (CAT) facilitate the treatment with restriction enzymes. Bases from the fourth base to ninth base from the 5'-end (GTC GAC) serve as a restriction enzyme Sal I site.)

### (b) Preparation of template DNA

cDNA of ZR75-1 cells derived from human breast cancer was used as the template to amplify the full-length cDNAs of cyclin and CDK by the PCR method. First, total RNA was extracted and purified from ZR75-1 cells by using phenol-thiocyanic acid guanidine method (Nippon Gene, Isogen) to prepare the cDNA. The cDNA was synthesized from the extracted total RNA, using oligo-dT or oligo-random primers (reverse transcription).

### (c) Condition of PCR

The PCR was carried out under the following conditions with three stages:
1) 1 cycle at 95°C for 5 min (denaturing), at 52°C for 1 min (annealing), and at 72°C for 2 min (extension);
2) 35 cycles at 94°C for 30 sec (denaturing), at 62°C for 30 sec (annealing), and at 72°C for 2 min (extension); and
3) 1 cycle at 72°C for 10 min.

### (d) Cloning into vectors and preparation of recombinant baculovirus solution

Each amplified PCR product was digested with corresponding restriction enzymes, respectively. After subjecting each DNA digested with the restriction enzyme to agarose gel electrophoresis, the digested products were purified on Geneclean (BIO101). After ligating the purified DNA to the pFASTBACHT vector (GIBCO BRL) that has been previously digested with the same restriction enzyme used for digesting both ends of the PCR products, *Escherichia coli* strain DH5α was transformed with the vector according to the method described in "Molecular Cloning (Sambrook et al., second edition (1989), Cold Spring Harbor Laboratory Press, Cold Spring Harbor, NY)", thereby obtaining plural single colonies. The purified pFASTBACHT vectors (pFASTBAC-n6his-hcyD, pFASTBAC-n6his-hcyE, pFASTBAC-n6his-hcyB, pFASTBAC-n6his-hCDK1, pFASTBAC-n6his-hCDK2, and pFASTBAC-n6his-hCDK4) were transformed into *Escherichia coli* strain DH10BAC, and the transformed bacteria were grown on a Luria agar plate (10 g/L peptone, 5 g/L yeast extract, 10 g/L NaCl, 12 g/L agar, 200 µg/ml X-Gal, 40 µg/ml IPTG, 10 µg/ml tetracycline, 7 µg/ml gentamycin, and 50 µg/ml kanamycin). The expression cassette in pFASTBACHT is translocated into bacmid DNA by site specific transposition in DH10BAC. Recombinant bacmid DNA was purified from the white single colonies wherein translocation occurred. Sf9 cells were transfected with recombinant bacmid DNA using CELL FECTIN (GIBCO BRL). Since the recombinant baculovirus was produced in the culture medium, the culture medium was centrifuged after about one week in order to use its supernatant as a virus solution. Most of the procedures above were carried out using the BAC-TO-BAC Baculovirus Expression System (GIBCO BRL).

### (e) Confirmation of the nucleotide sequence

pFASTBAC-n6his-hcyD, pFASTBAC-n6his-hcyE, pFASTBAC-n6his-hcyB, pFASTBAC-n6his-hCDK1, pFASTBAC-n6his-hCDK2, and pFASTBAC-n6his-hCDK4 were purified according to the method in "Molecular Biology" cited above. The nucleotide sequences of these insert DNAs were determined with an auto-sequencer based on the Sanger's method. It was confirmed that the cloned cDNAs were identical to cyclin D1, cyclin B, cyclin E, CDK1, CDK2, and CDK4, respectively, by comparing the sequences of the insert DNAs with the sequences disclosed in database.

### (f) Purification of the recombinant proteins

The Sf9 cells were cultured in a 75 cm² flask to an almost confluent state, and the recombinant baculovirus solution was added to the culture medium. 3 to 4 days after the infection, the cells were suspended in ice-cooled PBS, and were washed by centrifugation. The cells were then suspended in an HGN buffer (-DTT), and were subjected to ultrasonication with cooling on ice. Subsequently, the solution was centrifuged, and the supernatant obtained as a soluble fraction was used for purification. Six His residues (6x His) are added to the N-terminus in the expression cassette of the pFASTBACHT vector. A part of the extract was used for the Western blot analysis, and expression of the protein was confirmed using anti-5x His antibodies (QIAGEN). The soluble fraction was applied to a Ni-chelating Sepharose column (Pharmacia) to allow the protein to be adsorbed on the column via the 6x His-Tag. Since the 6x His-Tag forms a complex with Ni²⁺, the recombinant protein binds to Ni²⁺ on the column. After washing the column with the HGN buffer (-DTT), the recombinant proteins were eluted with an elution buffer (50 mM to 1 M imidazole, 0.5 M NaCl, 20 mM Tris-HCl, pH 7.9). The elution was performed with a stepwise imidazole concentration of 10 mM, 25 mM, 50 mM, 100 mM, 250 mM, 500 mM, and 1 M.

The recombinant proteins in each imidazole fraction were detected with the anti-5x His antibodies using the Western blot assay, and those fractions detected to contain the recombinant protein were dialyzed against a dialysis solution (50 mM HEPES (pH 7.5), 1 mM DTT, 1 mM EDTA (pH 8.0), 50% glycerol) overnight.

After the dialysis, the sample was applied to a MonoQ column (Pharmacia). The adsorbed protein was eluted using FPLC (Pharmacia) with an elution solution with a concentration gradient containing 0 to 1 M NaCl in 10 mM Tris-HCl (pH 7.5), 1 mM DTT, and 10% glycerol. The recombinant proteins in the fractions were detected with anti-5x His antibodies by the Western blot assay, and the fractions detected to contain the recombinant protein were dialyzed against the dialysis solution described above.

### [Example 3] Activity measuring systems for cyclin D1/CDK4, cyclin E/CDK2, and cyclin B/CDK 1 complex

### (1) Preparation of substrate for activity measurement

While various molecules are known as substrates for the cyclin/CDK complexes, an RB protein, which is most interesting among these proteins because of relations to carcinogenesis, was selected as the substrate. cDNA sequences encoding the 307th amino acid to the 405the amino acid (307aa-405aa), 540aa-645aa, and 723aa-833aa of the human RB protein amino acid sequence were introduced into an expression vector pGEX, and the sequences were expressed as fusion proteins with the glutathione-S-transferase (GST) in *Escherichia coli*. The expressed products were dubbed GST-pRB1, GST-pRB2, and GSTpRB3, respectively, in the order of the amino acid sequences above.

The recombinant protein was produced as a fusion protein with GST when the pGEX vector is used. The recombinant protein was purified by taking advantage of the fact that this GST enzyme has an affinity for glutathione (GSH). After confirming the expression of the recombinant protein, the cells were suspended in 1% Tween 20-PBS, and then were disrupted by ultrasonication. After high-speed centrifugation, the supernatant was taken as the soluble fraction containing the recombinant protein. The soluble fraction was passed through a GSH-Sepharose 4B column (Pharmacia) and GST-p53 (1-99) the protein was absorbed on the column. The column was washed with WE buffer (10 mM 2-mercaptoethanol, 2 mM MgCl₂, 20 mM Tris-HCl pH 7.5), and then, the recombinant protein was eluted with G buffer (10 mM GSH, 50 mM Tris-HCl pH 9.6). Each eluted expression protein was dialyzed against a dialysis solution containing 50 mM Tris-HCl(pH 8.0) and 50% glycerin.

### (2) Preparation of substrate sensitized plate

GST-pRB1, GST-pRB2, and GST-pRB3 were diluted with PBS to a concentration of 20 µg/ml, and a microtiter plate was sensitized at 4°C overnight after dispensing 50 µl of each of the diluted solutions into each well of the ELISA microtiter plate. After sensitization, the wells were blocked at room temperature for more than 1 hour after dispensing 200 µl of blocking solution containing 1% BSA and 0.1% Tween 20-PBS to each well.

### (3) Phosphorylation reaction

Respective enzymes (recombinant cyclin D1/CDK4, cyclin E/CDK2 or cyclin B/CDK1 complex) were added in a cyclin/CDK reaction buffer (50 mM HEPES, 15 mM MgCl₂, 1 mM DTT, 0.02% Triton X, 1 mM EGTA, 5 mM ATP) so that the quantity of the enzyme became about 100 ng per assay, and the enzyme solutions were dispensed to each well (50 µl/well) of a solid phase reaction plate. Then the plates were incubated at 30°C for 1 hour.

### (4) Antigen-antibody reaction

Primary reaction: After washing the wells of the plate with PBS five times, 100 µl of diluted (50-fold) anti-phosphorylated monoclonal antibodies T356 (1C2), S612 (3C12), S780 (2C4), and S812 (5H12) were dispensed to each well followed by incubation at room temperature for 1 hour.

Secondary reaction: After washing the wells of the plate with PBS 10 times, 100 µl of diluted (40,000-fold) HRP-labeled anti-mouse IgG (Immunotech Co.) was dispensed to each well followed by incubation at room temperature for 1 hour.

Color reaction: After washing the wells of the plate with PBS five times, 100 µl of TMB (tetramethylbentidine) solution was dispensed to each well. After leaving the reaction product to stand for 30 minutes to form colors at room temperature, the coloring reaction was stopped by adding 100 µl of 1.5N H₃PO₄ solution to each well.

### (5) Results of measurements

As shown in Table 2, the activity of the expressed cyclin/CDK complexes could be detected with high sensitivity.

**Table 2**

| Substrate Antibody | GST-pRB1 T356 | GST-pRB2 S612 | GST-pRB3 S780 | GST-pRB3 S807 |
|---|---|---|---|---|
| Complex | | | | |
| D1/CDK4 | 0.482 | 1.184 | 1.076 | 0.441 |
| E/CDK2 | 2.301 | 1.354 | 0.092 | 0.482 |
| B/CDK1 | 1.713 | 1.412 | 0.199 | 0.740 |
| no enzyme | 0.030 | 0.074 | 0.076 | 0.052 |

### [Example 4] Construction of cyclin D1/CDK inhibitor screening system

### 〈ELISA method〉

### 1) Sensitization conditions for antibodies

Clone 1C12 (specific to Thr356), clone 4E4 (specific to Ser612), and clone 2C4 (specific to Ser780) were used as the anti-phosphorylated RB monoclonal antibodies. Each anti-phosphorylated RB monoclonal antibody was diluted to a concentration of 10 µg/ml in PBS, and 100 µl of the diluted solution was added to each well of the plate followed by incubating at 4 °C for 15 hours. After removing the antibody solution, the reaction product in each well was blocked at 4°C for 24 hours in a PBS solution (5% sucrose, 0.1% NaN₃) containing 2.5% of BSA. The plate was subjected to measurements after airdrying.

### 2) Phosphorylation reaction

The RB amino acid sequence 301aa-928aa was expressed as a GST fusion protein in *Escherichia coli*, and the protein (RB301) purified using glutathione Sepharose and phosho-Cellulose columns (P11) was used as the substrate. Cyclin D1/CDK4, cyclin A/CDK2, cyclin E/CDK2, and cyclin B/CDK1, respectively, were expressed in the sf9 cells using baculovirus, and were purified using a cobalt column and phospho-Cellulose column in order to use them as the cyclin/CDK complexes.

RB301 (10µg/ml), 100µM ATP, and respective Cyclin/CDK were added to the phosphate buffer (50 mM HEPES, 0.05% Triton X, 15 mM MgCl₂, 2 mM EGTA, 200 µg/ml BSA) (final reaction volume of 180 µl), and was reacted for 30 minutes at 30°C. The reaction was stopped by adding 180 µl of PBS (10% skim milk, 350 mM NaCl, 50 mM EDTA) to each assay system.

### 3) Reaction

After completing the phosphorylation reaction, 100 µl of each of the reaction solutions was added to the antibody sensitization plate, which was incubated at room temperature for 1 hour (primary reaction). After washing with PBS [washing solution: PBS (500 mM NaCl, 0.1% Tween 20)] five times using a wash-bottle, 100 µl of anti-GST polyclonal antibody solution (0.5 µg/ml) was added to each well of the microplate, followed by incubation at room temperature for 1 hour (secondary reaction). After washing with PBS [washing solution: PBS (500 mM NaCl, 0.1% Tween 20)] five times using a wash-bottle, 100 µl of HRP labeled anti-rabbit IgG antibody solution (20,000-fold dilution) was added to each well of the microplate, followed by incubation at room temperature for 30 minutes (tertiary reaction). Then, the reaction solution was colored (coloring reaction) at room temperature for 30 minutes using TMB (tetramethylbentidine).

### 〈RI method〉

After a phosphorylation reaction with 0.3 µCi γ-³²P-ATP by the same ELISA method as described above, the reaction was stopped with 10% TCA and 0.2% sodium pyrrophosphate. The insoluble substrate was adsorbed on a glass filter, washed with 2% TCA and 0.02% sodium pyrrophosphate to count the radiation dose.

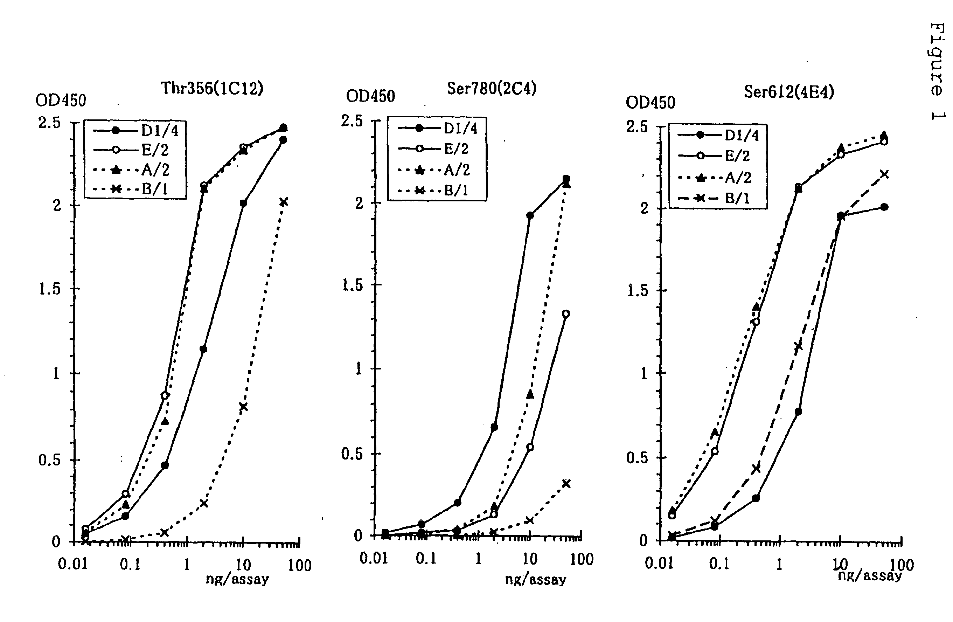

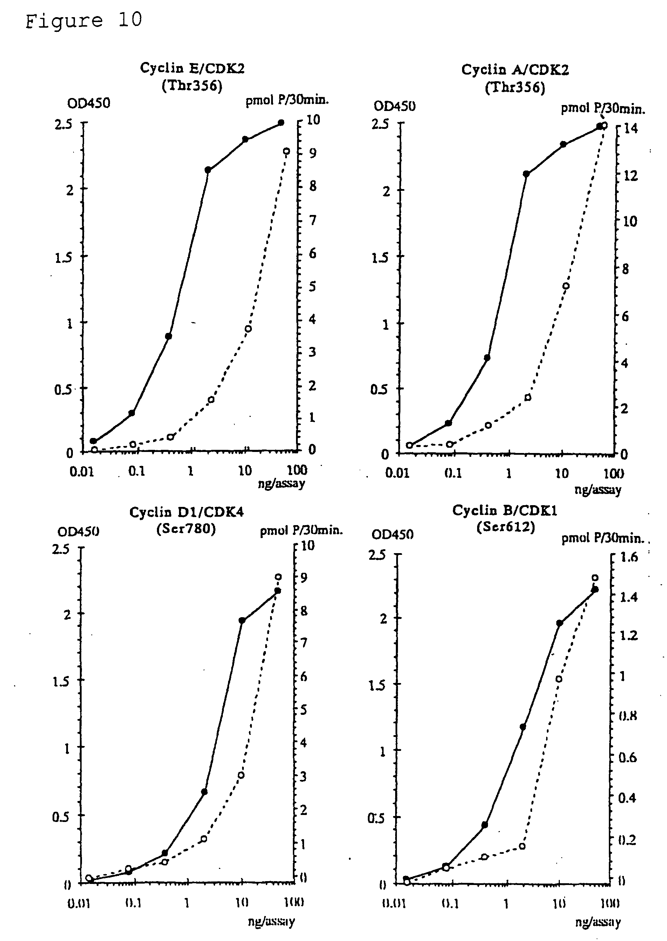

Figure 1 shows the results of the phosphorylatioh reaction measurements by the ELISA method. An enzyme amount dependent increase in the OD value was observed in all the combinations of the enzymes and phosphorylation sites, and the reaction curve showed a smooth sigmoid curve. This result suggested that the ELISA system constructed in the present invention could be used favorably to measure the phosphorylation reaction of the cyclin/CDK complex. It was also shown that the phosphorylation site of each enzyme was selective from the comparisons of the phosphorylation activity at each site among the four kinds of purified enzymes. Cyclin A/CDK2 and cyclin E/CDK2 were selective to Thr356, while cyclin D1/CDK4 was selective to Ser780. Cyclin B/CDK1 showed selectivity to Ser612. The sensitivity of the ELISA method was shown to be identical or even more sensitive to that of the RI method according to a comparison between these two methods (Fig. 10). Particularly, the sensitivity of the ELISA method using cyclin A/CDK2 and cyclin E/CDK2 was determined to be 10-fold higher than the sensitivity of the RI method.

Next, the possibility of assessing the inhibitory effect of the inhibitor using the ELISA system according to the present invention was investigated using commercially available reagents.

Five kinds of reagents, Olomoucine (IC50 = 7 µM) and Roscovitine (IC50 = 700 nM) which are selective inhibitors to cdc2 and cdk2, respectively; K252a (Ki = 25 nM against PKC) as a general inhibitor of phosphorylation enzymes; U0126 (IC50 = 55 nM; > 10µM against CDK2 and CDK4) which is an inhibitor to MEK; and Olomoucine-isomer (a negative control to Olomoucine), were used as the reagents.

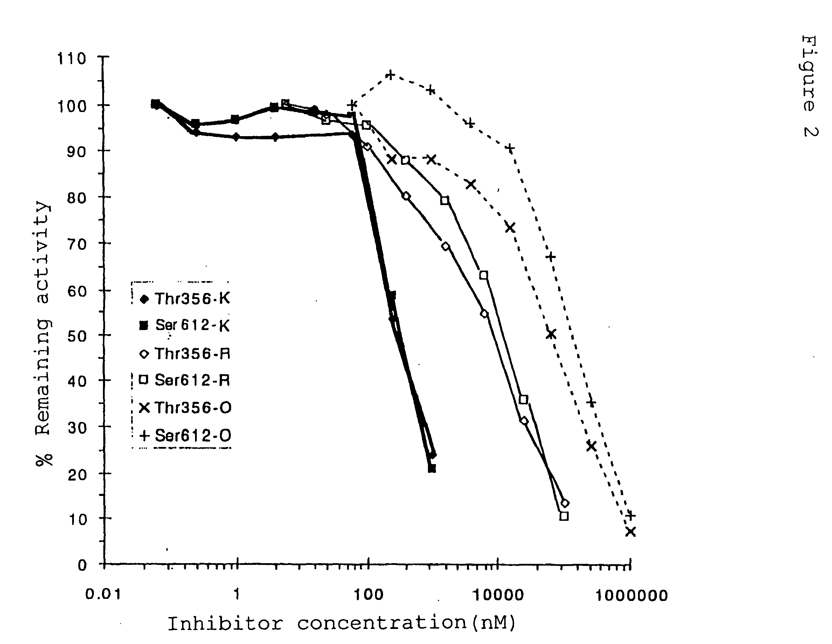

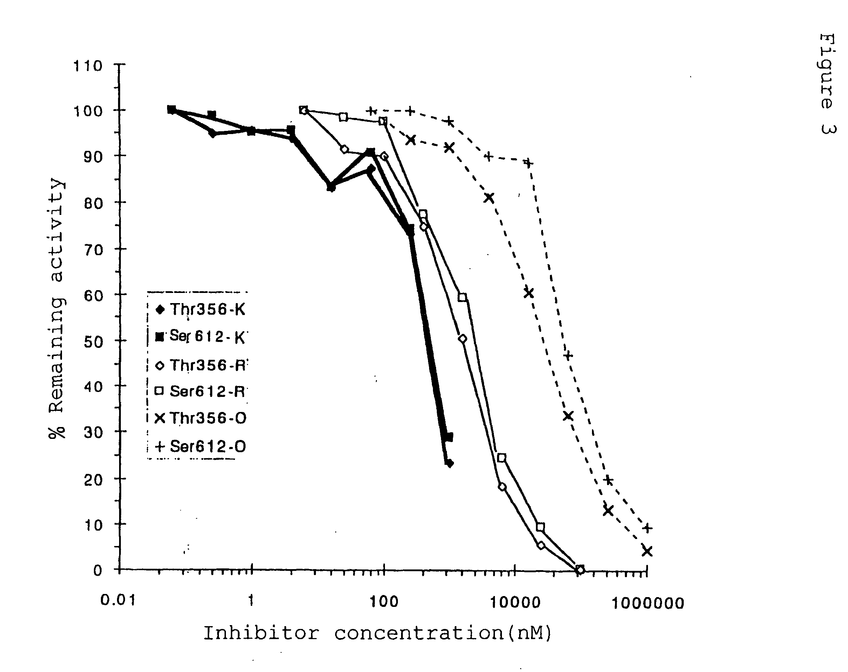

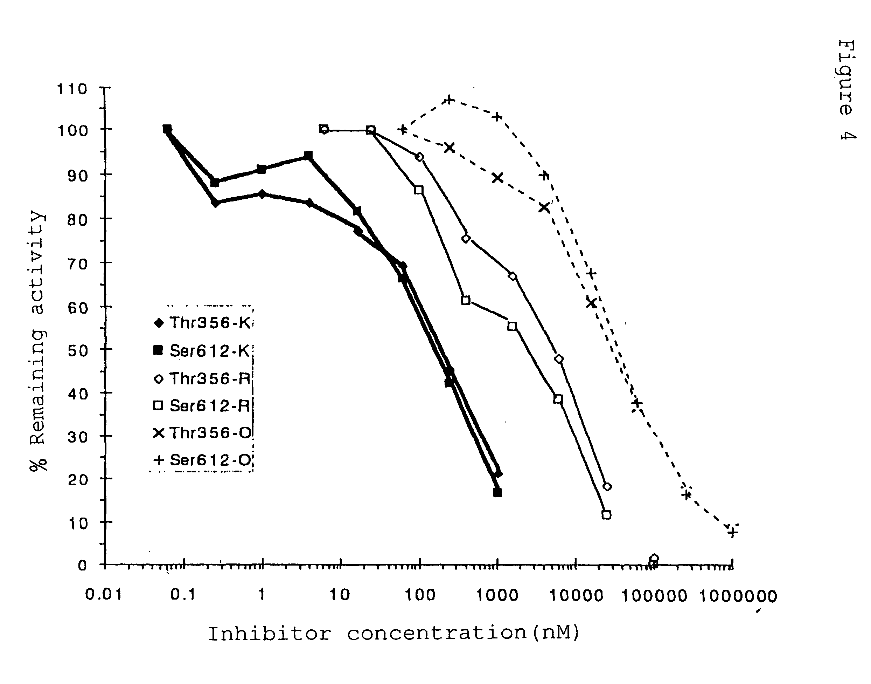

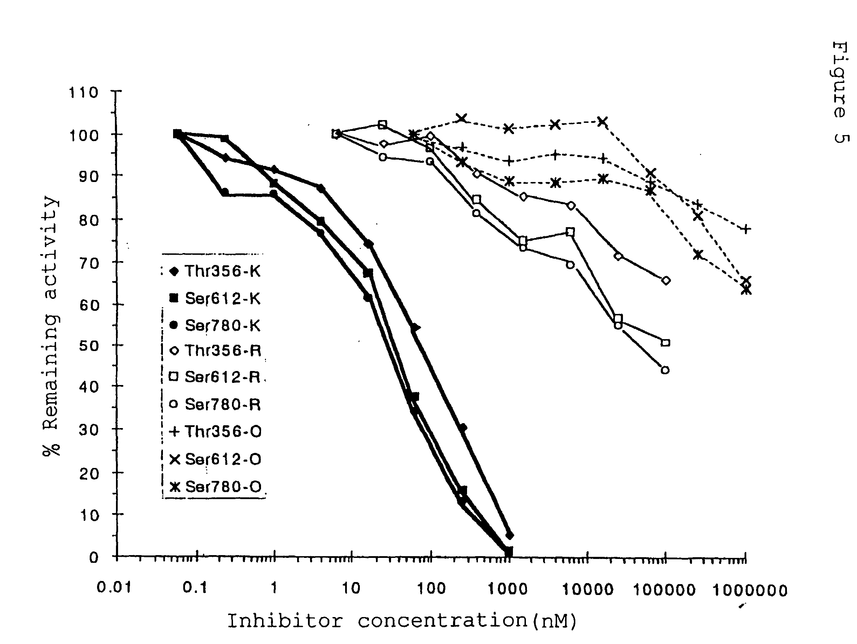

Figures 2 to 5 show the results of investigations on the inhibitory effects of K252a, Olomoucine, and Roscovitine by the ELISA method. The OD levels decreased in a inhibitor concentration-dependent manner with any of the inhibitors. While IC50 of Olomoucine and Roscovitine are 7 µM and 700 µM, respectively, at their nominal levels, they were about 10-fold higher than these levels according to the evaluation in the present invention.

The difference above was presumed to be due to the difference in ATP concentration used in the assay system, since these inhibitors also act as ATP antagonists. Another cause of the difference was the ATP concentration and the used substrate was 15 µM and histone H1, respectively, in the literature ("Inhibition of cyclin-dependent kinases by purine analogues", Vesely J., Havliek L., Stand M., Blow JJ., Donella-Deana A., Pinna L., Letham DS., Kato J., Detivaud L., Leclerc S. et al, Eur. J. Biochem. 1994, Sep. 1, 224:2, 771-86), whereas those used in this ELISA system was 100 µM and RB protein, respectively.

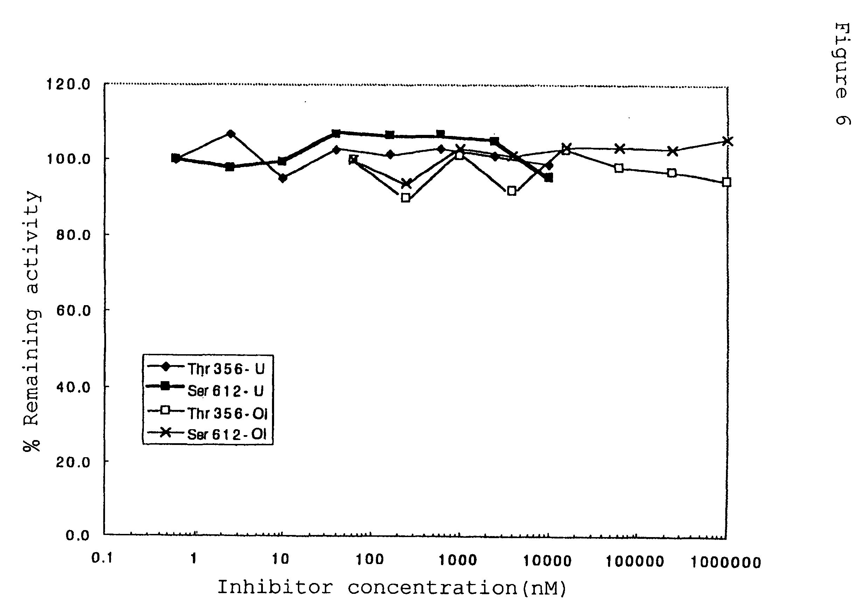

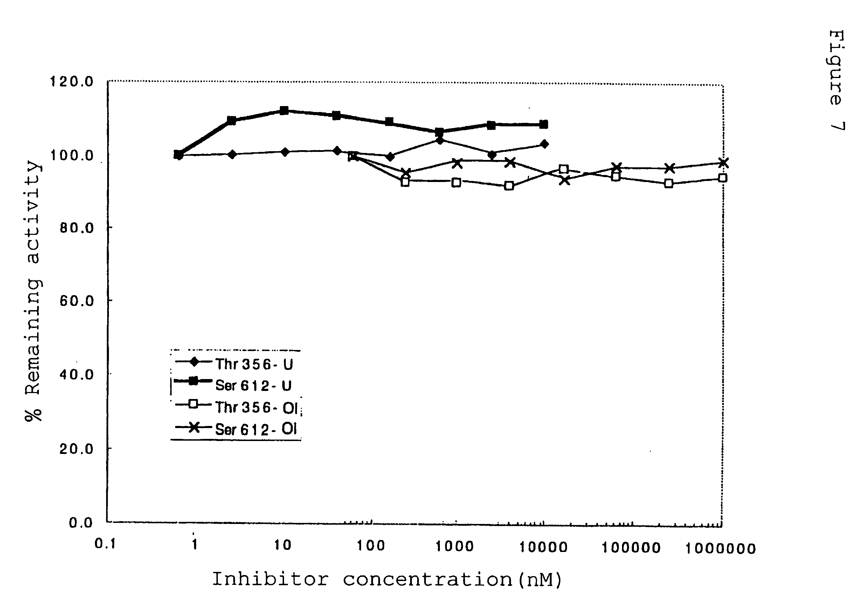

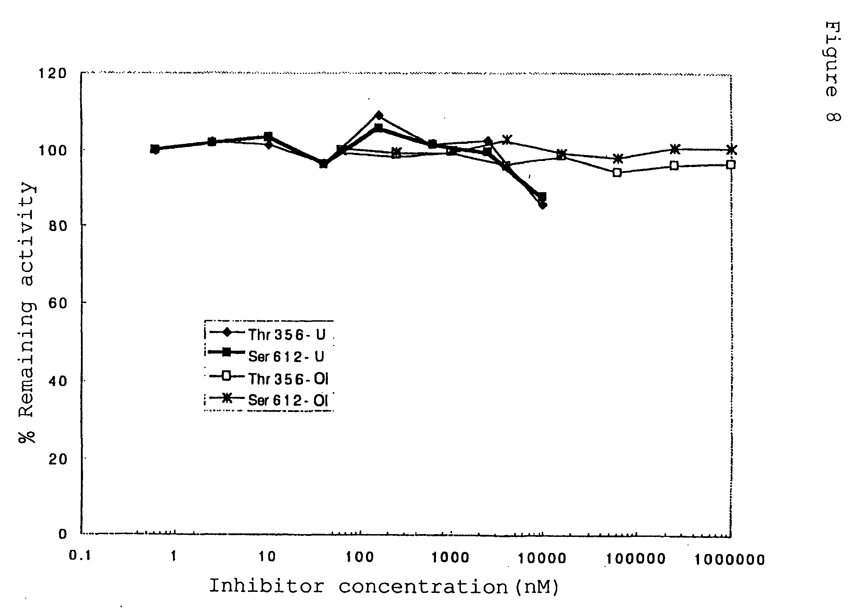

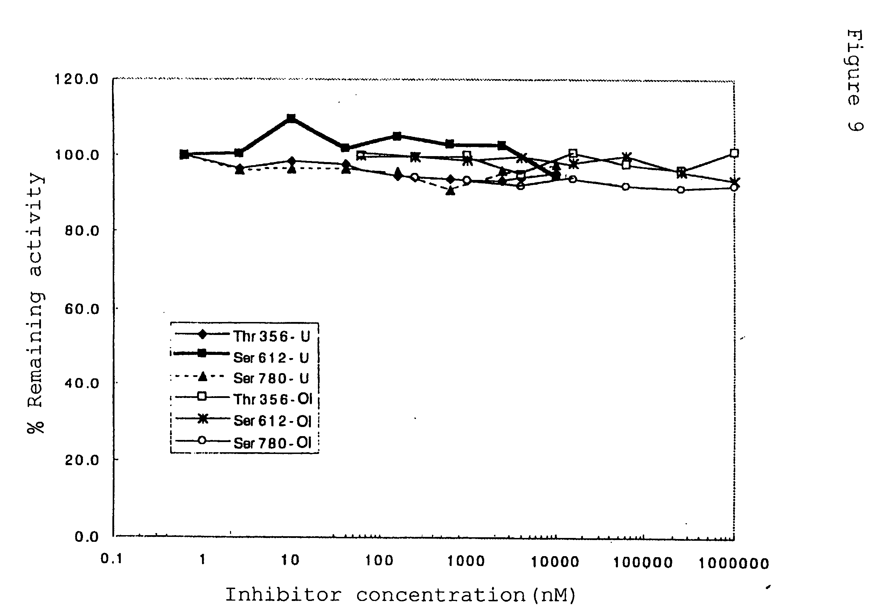

It was also reported that the Olomoucine and Roscovitine are selective inhibitors for cdc2 and cdk2, respectively, but show remarkably weak inhibitory effect on cdk4 ("Biochemical and cellular effect of roscovitine, a potent and selective inhibitor of the cyclin-dependent kinases cdc2, cdk2 and cdk5", Meijer L., Borgne A., Mulner O., Chong JP., Blow JJ., Inagaki M., Delcros JG. Moulipoux JP., Eur. J. Biochem 1997, Jan 15 243:1-2, 527-39). The experiment results of the present invention reflected this report (the inhibitory effect on cyclin D1/CDK4 is weaker than those on the other three cyclin/CDK complexes). Furthermore, almost no decrease in the OD value was observed in the concentration range investigated for Olomoucine-isomer and U0126, which couldn't be expected to have inhibitory effects (Figs. 6 to 9).

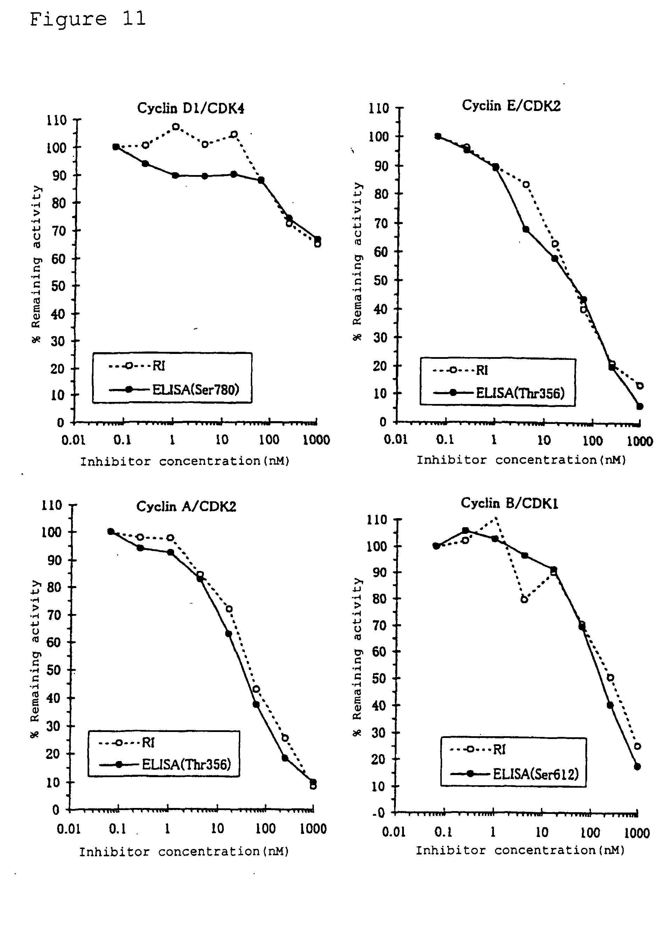

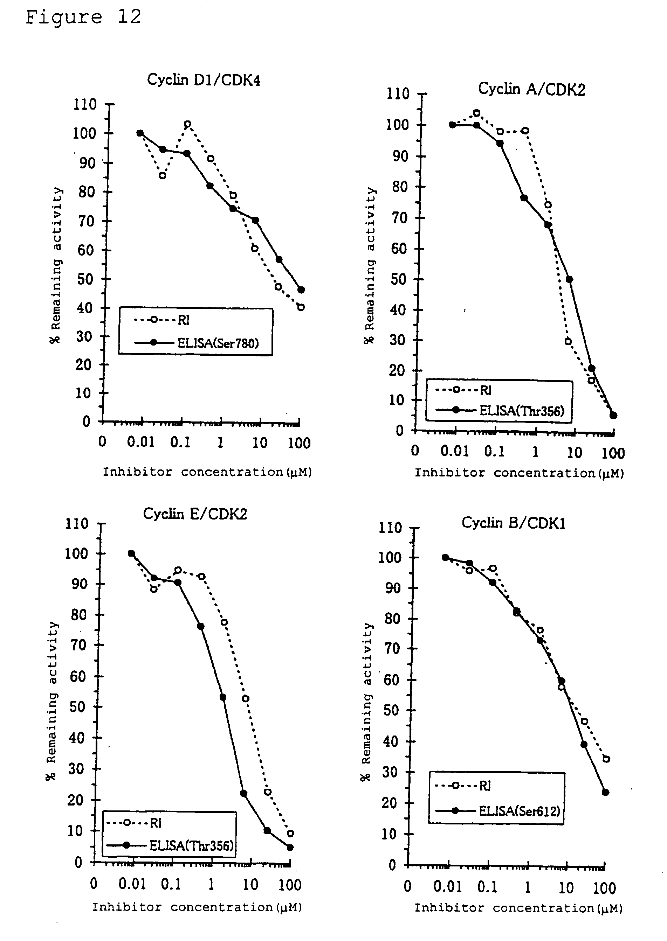

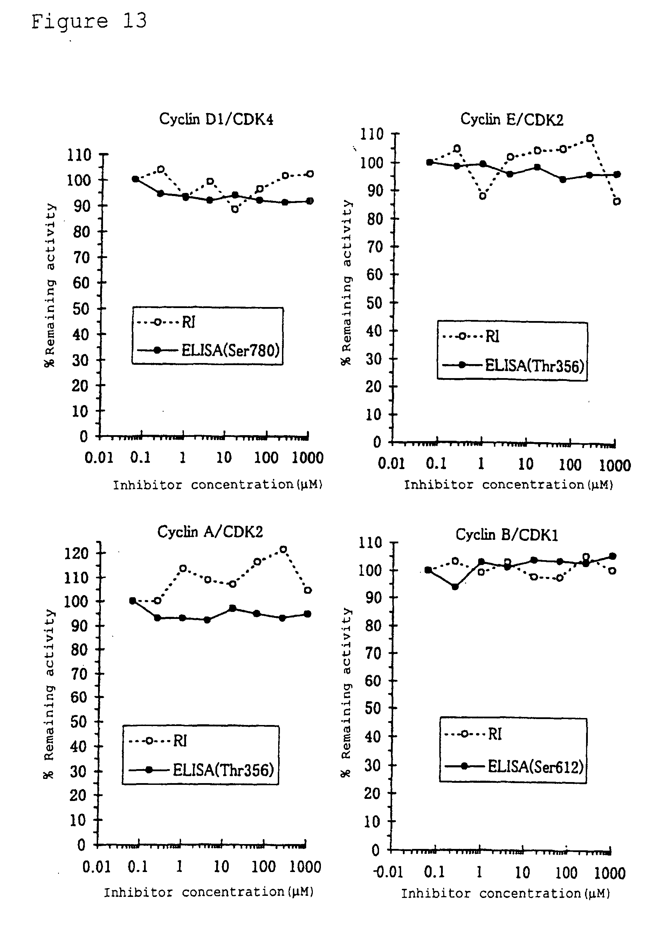

The assessment by the ELISA method and RI method on the inhibitory effects of Olomoucine, Roscovitine, and Olomoucine-isomer were compared to each other, in order to determine whether the inhibitory effect can be assessed by the ELISA method as well as by the RI method. As a result, almost the same inhibitory curves could be obtained by both of the two methods, as shown in Figs. 11 to 13.

It was confirmed from the discussions above that the ELISA system constructed in the present invention is useful as a method of screening for inhibitors.

### Industrial Applicability

As shown in the examples, enzyme activities of cyclin D1/CDK4, cyclin E/CDK2, and cyclin B/CDK1 can be measured by the ELISA method utilizing anti-phosphorylated peptide-specific antibodies. In the past, special procedures for disposal of waste and special facilities were required, since γ-³²P-ATP labeled with radioactive ³²P was typically used in the activity measurement of these enzymes. In contrast, measuring methods with sufficiently high sensitivity can be realized without using RI in the method according to the present invention. Furthermore, only the activity of a selected complex can be specifically determined in a sample containing plural cyclin/CDK complexes according to the present invention by utilizing antibodies specifically recognizing the phosphorylation site.

A simple and rapid screening can be accomplished in a standard laboratory, by applying this measuring method to the screening of an activity regulating agent of a cyclin/CDK complex. In addition, the screening method according to the present invention is applicable to a wide range of candidate compounds, such as various kinds of synthetic chemicals, extracts of cells and bacteria, and culture medium supernatant. Accordingly, the method according to the present invention is very useful for screening compounds that regulate the enzyme activity of a cyclin/CDK complex.

## Claims

1. A method of measuring a kinase activity of a cyclin/CDK complex in a sample on RB proteins or partial peptides thereof, comprising the steps of:
(a) contacting a substrate comprising the RB protein with the sample under conditions required for the phosphorylation reaction associated with the kinase to occur; and
(b) detecting changes in the phosphorylation level of the RB protein substrate based on changes in the reactivity of the substrate with antibodies that identify the phosphorylation state of the substrate.

2. A method of screening for compounds that inhibit or enhance the kinase activity of a cyclin/CDK complex on an RB protein, comprising the steps of:
(a) contacting and incubating the cyclin/CDK complex with a substrate comprising the RB protein in the presence of a test compound under conditions required for the phosphorylation reaction to occur;
(b) detecting changes in the phosphorylation level of the RB protein substrate based on changes in the reactivity of the substrate with antibodies that identify the phosphorylation state of the substrate; and
(c) selecting the compound that lowers or elevates the phosphorylation level of the substrate compared to the levels detected in the absence of the test compound.

3. A method of measuring a phosphatase activity on phosphorylated RB proteins in a sample, comprising the steps of:
(a) contacting and incubating a substrate comprising the phosphorylated RB protein with the sample under conditions required for the dephosphorylation reaction to occur; and
(b) detecting changes in the phosphorylation level of the RB protein substrate based on changes in the reactivity of the substrate with antibodies that identify the phosphorylation state of the substrate,
wherein the phosphorylated RB protein is phosphorylated by a cyclin/CDK complex.

4. A method of screening for compounds that inhibit or enhance the phosphatase activity on phosphorylated RB proteins, comprising the steps of:
(a) contacting and incubating the phosphatase against the phosphorylated RB protein with a substrate comprising the phosphorylated RB protein in the presence of a test compound under conditions required for the dephosphorylation reaction to occur;
(b) detecting changes in the dephosphorylation level of the RB protein substrate based on changes in the reactivity of the substrate with antibodies that identify the phosphorylation states of the substrate; and
(c) selecting a compound that lowers or elevates the dephosphorylation level of the substrate compared to the levels detected in the absence of the test compound.

5. The measuring method of any one of Claims 1 to 4, wherein the cyclin is selected from the group consisting of: cyclin A, cyclin B, cyclin D1, cyclin D2, cyclin D3, and cyclin E.

6. The measuring method of any one of Claims 1 to 4, wherein the CDK is selected from the group consisting of: CDK 1, CDK 2, CDK 4, and CDK 6.

7. The measuring method of any one of Claims 1 to 4, wherein the cyclin/CDK complex comprises any one of the combinations selected from the group consisting of:
cyclin A/CDK1;
cyclin A/CDK2;
cyclin B/CDK1;
cyclin D1/CDK4;
cyclin D1/CDK6;
cyclin D2/CDK4;
cyclin D2/CDK6;
cyclin D3/CDK4;
cyclin D3/CDK6; and
cyclin E/CDK2.

8. The method of Claim 7, wherein the kinase is the cyclin D1/CDK4 complex, and the antibody identifies the phosphorylation state of the serine at position 780 of the RB protein.

9. The method of Claim 1, wherein the individual activities of the cyclin/CDK complexes contained in a sample comprising a plurality of cyclin/CDK complexes that separately phosphorylates different sites can be measured, wherein the antibody that identifies the phosphorylation state identifies the phosphorylation site.

10. The method of any one of Claims 1 to 4, wherein the antibody has a higher reactivity with the phosphorylated RB protein substrate than with a non-phosphorylated RB protein substrate.

11. The method of any one of Claims 1 to 4, wherein the antibody has a higher reactivity with a non-phosphorylated RB protein substrate than with the phosphorylated RB protein substrate.

12. The method of any one of Claims 1 to 4, wherein the substrate is labeled.

13. The method of any one of Claims 1 to 4, wherein substrate is immobilized on a solid phase.

14. The method of any one of Claims 1 to 4, wherein the antibody is labeled.

15. The method of any one of Claims 1 to 4, wherein the phosphorylation level of the substrate is evaluated by the ELISA method.

16. A protein kinase activity regulating agent of the cyclin/CDK complex containing a compound, which can be isolated by the screening method described in Claim 2 as the active ingredient.

17. A protein phosphatase activity regulating agent of a protein phosphorylated by the kinase of a cyclin/CDK complex containing a compound, which can be isolated by the screening method described in Claim 4 as the main principle.

18. The activity regulating agent of Claim 16 or 17, wherein the agent is derived from nature.

19. An immunogen for preparing antibodies used in any of the methods of Claims 1 to 4, containing the RB protein or a peptide comprising an amino acid sequence including one or more phosphorylation sites of the homologues of the RB protein in species other than humans.

20. The immunogen of Claim 19, wherein the phosphorylation site(s) is(are) phosphorylated.

21. The immunogen of Claim 19 or 20, wherein the phosphorylation site is at least one site selected from the group consisting of: threonine at position 356; serine at position 612; serine at position 780; and threonine at position 807 of the human RB protein.

22. The immunogen of Claim 21, wherein the phosphorylation site is selected from the group of amino acid sequences consisting of the sequence shown in SEQ ID NOs: 2, 4, 6, and 8.

23. A kit for performing the methods of Claims 1 to 4, wherein the kit contains an antibody that identifies the phosphorylation state of the substrate.
